# EUROPEAN PATENT APPLICATION

(11) **EP 4 000 682 A1**
(43) Date of publication of application: **25.05.2022**
(21) Application number: 19937805.0
(22) Date of filing: 18.10.2019
(51) Int. Cl.: A61N 1/36, A61N 1/40, A61N 1/32, A61N 1/05, A61N 1/06, A61N 1/04

(54) **NEEDLE TIP FOR APPLYING CURRENT, HANDPIECE, AND SKIN TREATING DEVICE**

(30) Priority: 17.07.2019 KR 20190086629
(71) Applicant: Jeisys Medical Inc., Geumcheon-gu Seoul 08501 (KR)
(72) Inventor: KANG, Dong Hwan, Incheon 22229 (KR); SUN, Hye Jin, Incheon 22120 (KR); CHO, Seung Hwan, Seoul 03469 (KR)
(74) Representative: Becker Kurig & Partner Patentanwälte mbB
(86) International application number: PCT/KR2019/013705
(87) International publication number: WO 2021/010546

(57) **Abstract**

A needle tip for applying current is provided. The needle tip for applying current includes a needle fixing unit, and a plurality of needles disposed on one surface of the needle fixing unit, wherein each needle includes at least one active region which is formed, as an insulator is not coated on a partial area of the needle except for a distal end portion of the needle and electromagnetically conducted. The plurality of needles are connected to electrodes having alternately different polarities. When the plurality of needles include the plurality of active regions spaced apart from each other, the intensity of current applied to each of electrodes connected to the plurality of needles is adjusted, such that electric energy is transmitted between the plurality of active regions spaced apart from each other.

## Description

### [TECHNICAL FIELD]

Embodiments of the inventive concept described herein relate to a needle tip for applying current, a handpiece, and an apparatus for treating skin, in which the needle tip includes a needle having an active region formed at a partial region of the needle except for a distal end portion of the needle to be uninsulated for electromagnetic conduction.

### [BACKGROUND ART]

In general, skin functions as a primary barrier to prevent environmental influences such as sunlight, cold, and wind. With aging, the skin loses the vital appearance due to environmental influences and acquires wrinkles.

The human skin includes an epidermis layer having a thickness of 100 µm, a dermis layer positioned under the epidermis layer and formed to the depth of 4 mm from the skin surface, and a subcutaneous layer. The three layers determine the overall appearance of skin. The dermis layer includes elastin collagen, glycosaminoglycan, and proteoglycan. The subcutaneous layer has a fibrous vertical band that crosses the dermis layer and connects the dermal collagen to the subcutaneous layer. Collagen fibers provide rigidity and elasticity to the skin.

However, collagen may lose elasticity due to aging and exposure to sunlight, which causes people to lose their young and vivid appearance. Accordingly, many manners have been suggested to revitalize the skin. In general, a manner of treating skin for the purposes of treating various scars or skin diseases, or for the cosmetic purpose of skin improvement or wrinkle care is to solidify tissues by applying various energy sources to a target part. For example, manners for transmitting thermal energy have been known.

According to the manners, various types of energy are applied to a target part of the skin to intentionally cause wounds and to simulate collagen in the dermis layer, thereby regenerating the target part of the skin by inducing the regeneration of collagen. Relevant papers say that collagen denaturation is most reliably induced at the temperature of 65 °C to 75 °C..

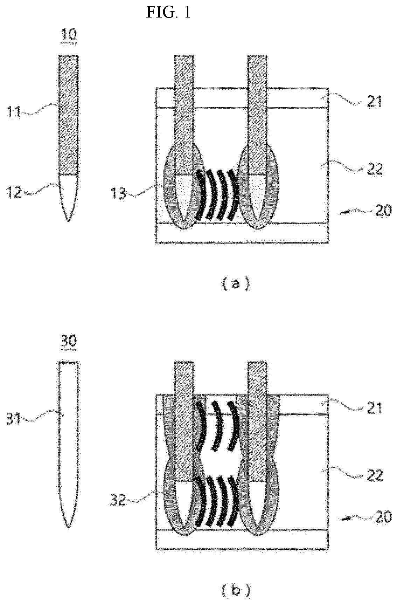

Referring to FIG. 1A and FIG. 1B, conventionally, there has been employed a manner to apply electric energy (RF) to a treatment part of a skin 20 by injecting an insulating needle 10 illustrated in FIG. 1A or a non-insulating needle 30 illustrated in FIG. 1B into an epidermis 21 and a dermis 22 of the skin 20. However, as energy is intensively applied to a needle distal end due to the characteristic in which electric energy is intensively applied to a pointed end, electric energy is intensively applied to the distal end portion in in the case of the insulating needle 10 having the entire portion coated with an insulator 11 except for a distal end portion 12 and in the case of the non-insulating needle 30. Accordingly, excessive lacerations are caused only in a part of the skin 20 in which the distal end portion is positioned.

In addition, the insulating needle 10 can transmit electric energy only to the skin 20 in which the distal end portion 12 is positioned, but cannot transmit the electric energy to the skin 20 positioned around the side surface of the needle.

In addition, although the non-insulating needle 30 can transmit electric energy even to the side surface of the non-insulating needle 30 because the entire portion 31 of the non-insulating needle 30 is not coated with an insulator, the electric energy is transmitted to all faces in contact with the needle. Accordingly, the energy is totally and excessively increased. In addition, as described above, even in the non-insulating needle 30, the electric energy may be intensively applied to the distal end portion, which causes undesirable pain.

In addition, the non-insulating needle 30 may transmit electric energy to all parts in which the needle is injected but cannot transmit electric energy only to any specific depth of the skin 20. Accordingly, the non-insulating needle 30 cannot treat only the skin 20 corresponding to the specific target depth.

### [DETAILED DESCRIPTION OF THE INVENTION]

### [TECHINICAL PROBLEM]

Embodiments of the inventive concept provide a needle for applying current, a handpiece, and an apparatus for treating skin, capable of transmitting electric energy only to skin parts, which are positioned at mutually different depths, to treat a specific skin, such that an undesired skin tissue is not damaged.

Embodiments of the inventive concept provide a needle for applying current, a handpiece, and an apparatus for treating skin, capable of preventing electric energy from being intensively applied to a distal end portion of a needle.

The objects of the inventive concept are not limited to the above, but other effects, which are not mentioned, will be apparently understood to those skilled in the art.

### [TECHNICAL SOLUTION]

According to an embodiment of the inventive concept, a needle tip for applying current includes a needle fixing part, and a plurality of needles disposed on one surface of the needle fixing unit, wherein each needle includes at least one active region which is formed, as an insulator is not coated on a partial area of the needle except for a distal end portion of the needle and electromagnetically conducted, and wherein the plurality of needles are connected to electrodes having alternately different polarities.

In addition, according to an embodiment, when the plurality of needles include a plurality of active regions spaced apart from each other, electric energy is transmitted between the plurality of active regions spaced apart from each other by adjusting intensity of current applied to each of the electrodes connected to the plurality of needles.

In addition, according to another embodiment, the needle tip further includes a casing making contact with skin surface to form a first space between the skin surface and the needle fixing unit and to move to allow the needle fixing unit to inject the needle into skin and discharge the needle out of the skin. The first space has a negative pressure formed before the needle is injected into the skin.

In addition, according to another embodiment, the needle tip is connected to a handpiece which moves the needle fixing unit and used. The active regions of the plurality of needles, which is electromagnetically conducted, is determined in position in skin by the control of a driving unit provided in the handpiece.

In addition, according to another embodiment, the plurality of needles include at least one active region, which is electromagnetically conducted, has an equal size, and is positioned at the same position of the needles except for the distal end portion of each needle. The needle tip heats skin tissue at a depth in which the active region is disposed, as the needle is injected into the skin.

In addition, according to another embodiment, the plurality of needles include a plurality of active regions formed at the same positions of the needles, except for distal end portions of the needles and having an equal size. The needle tip heats a tissue in a region of the skin, in which the plurality of active regions are disposed, and a tissue in an entire area of the skin, which is interposed between the plurality of active regions electromagnetically conducted, as the needle tip is injected into the skin and current having a specific intensity or more is applied.

In addition, according to another embodiment, a needle adjacent to a needle, which is connected to an electrode having a positive polarity, of the plurality of needles is connected to an electrode having a negative polarity.

According to another embodiment of the inventive concept, the apparatus for treating skin includes a casing to suction a target part of skin surface, a plurality of needles disposed inside the casing, a needle fixing unit to fix the plurality of needles inserted, a controller to control an operation of the casing and operations of the plurality of needles, and an electric energy transmitting unit electrically connected to the plurality of needles to transmit electric energy, and an electric energy generating unit to generate the electric energy to be transmitted to the plurality of needles of claim 1 through the electric energy transmitting unit.

In addition, according to another embodiment, a method for manufacturing a needle tip for applying current includes preparing a conductive material having a thickness for forming an active region corresponding to a non-insulated part and electromagnetically conducted, coupling a plurality of needles to the needle fixing unit, inserting the plurality of needles into the conductive material to positions of the plurality of needles, which are used for forming an active region electromagnetically conducted, and forming a distal end portion and an insulating area by spraying an insulating material in the status that the plurality of needles are inserted into the conductive material.

### [ADVANTAGEOUS EFFECTS OF THE INVENTION]

As described above, the inventive concept has the following effects.

According to the inventive concept, the electric energy may be transmitted only to the skin part positioned at a specific depth of the skin, based on the active region electromagnetically conducted.

In addition, according to the inventive concept, as the plurality of active regions electromagnetically conducted are formed, the electric energy may be simultaneously transmitted to skin parts adjacent to the active regions.

Further, according to the inventive concept, the length of the needle injected into the skin is adjusted to adjust the depth of the skin in which the active region of the needle is disposed.

In addition, according to the inventive concept, the electric energy may be prevented from being intensively applied to the distal end portion of the needle, thereby preventing undesirable pain of the patient having the skin complaint.

In addition, according to the inventive concept, the electric energy is transmitted only to the depth of the skin requiring the treatment, thereby preventing the unnecessary tissue of the skin from being heated and preventing the electric energy from being unnecessarily wasted.

### [DESCRIPTION OF THE DRAWINGS]

FIG. 1A and FIG. 1B are views illustrating a needle used in a conventional apparatus for treating skin;
FIG. 2 is an exploded perspective view illustrating an apparatus for treating skin, according to an embodiment of the inventive concept;
FIG. 3 is a cross-sectional view illustrating an apparatus for treating skin illustrated in FIG. 2;
FIG. 4 is a view illustrating a needle, according to an embodiment of the inventive concept;
FIG. 5 is a view illustrating an arrangement, based on a polarity of a needle, according to an embodiment of the inventive concept;
FIG. 6 is a view illustrating electric energy transmitted to skin by a needle, according to an embodiment of the inventive concept;
FIG. 7 is a view illustrating a needle suitable for each skin depth, according to an embodiment of the inventive concept;
FIG. 8 is a view illustrating an effect of transmitting electric energy by a needle, according to an embodiment of the inventive concept;
FIG. 9 is a perspective view illustrating a handpiece, according to an embodiment of the inventive concept;
FIG. 10 is a cross-sectional view of a needle tip for applying current, according to an embodiment of the inventive concept; and
FIG. 11 is a cross-sectional view of a needle tip for applying current, which operates to produce a pumping effect, according to an embodiment of the inventive concept.

### [BEST MODE]

### [INDUSTRIAL APPLICABILITY]

Hereinafter, an exemplary embodiment of the inventive concept will be described with reference to accompanying drawings. Advantage points and features of the prevent invention and a method of accomplishing thereof will become apparent from the following description with reference to the following figures, wherein embodiments will be described in detail with reference to the accompanying drawings. The inventive concept, however, may be embodied in various different forms, and should not be construed as being limited only to the illustrated embodiments. Rather, these embodiments are provided as examples so that this disclosure will be thorough and complete, and will fully convey the inventive concept to those skilled in the art. The inventive concept may be defined by scope of the claims. Meanwhile, the terminology used herein to describe embodiments of the invention is not intended to limit the scope of the inventive concept. Like reference numerals refer to like elements throughout the whole specification.

Unless otherwise defined, all terms (including technical and scientific terms) used herein have the same meaning as commonly understood by those skilled in the art. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The terminology used herein is provided for explaining embodiments, but the inventive concept is not limited thereto. As used herein, the singular terms "a," "an" and "the" are intended to include the plural forms as well, unless the context clearly indicates otherwise. Furthermore, it will be further understood that the terms "comprises", "comprising," "includes" and/or "including", when used herein, specify the presence of stated elements, steps, operations, and/or devices, but do not preclude the presence or addition of one or more other components, steps, operations and/or devices.

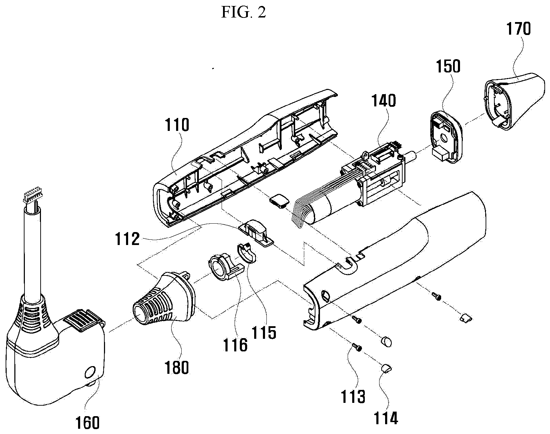

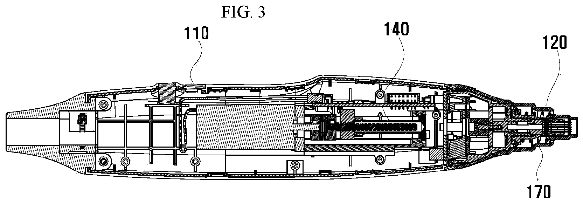

FIG. 2 is an exploded perspective view illustrating an apparatus for treating skin, according to an embodiment of the inventive concept. FIG. 3 is a cross-sectional view illustrating the apparatus for treating skin illustrated in FIG. 2.

Referring to FIGS. 2 to 3, according to an embodiment of the inventive concept, the apparatus for treating the skin may include a casing 110, a needle 120, a driving unit 140, an electricity supply unit 150, a connector 160, a cartridge 170, and a connection member 180.

According to an embodiment, after the surface of the cartridge 170 sticks to the skin, the cartridge 170 directly adjusts pressure at an inner part or changes the inner part (that is, the space between the skin surface and the needle fixing unit) of the cartridge 170 to be in a negative pressure status according to movement of the needle fixing unit. As the needle fixing unit is moved, the inner part (that is, the space between the skin surface and the needle fixing unit) of the cartridge 170 is changed to be in a negative pressure status, Accordingly, and the inner part of the cartridge 170 in the vacuum status suctions and lifts the skins due to the negative pressure, such that the target part to be treated is lifted.

In addition, according to another embodiment, the cartridge 170 sets skin region to be injected with needle 120. In addition, the cartridge 170 suctions a target part of skin surface to be injected with the needle 120, as the cartridge 170 includes only a wall to set a region for injecting the needle 120. Accordingly, an active region for the needle 120 is prevented from failing to be formed in an exact position as the skin surface is pressed when the needle 120 is injected. Accordingly, the target part is lifted, and the skin surface is flat, such that the needle 120 is injected.

According to an embodiment, after the surface of the cartridge 170 sticks to the skin, the cartridge 170 directly adjusts pressure at an inner part or changes the inner part (that is, the space between the skin surface and the needle fixing unit) of the cartridge 170 to be in a negative pressure status according to movement of the needle fixing unit Accordingly, the inner part of the cartridge 170 in the vacuum status suctions and lifts the skins due to the negative pressure, such that the target part to be treated is lifted.

In addition, according to an embodiment, the surface (i.e., an area making contact with the skin surface) of the cartridge 170 may be formed of a rubber material or silicone material to be in close contact with the target part of the skin. It is preferred that the cross-sectional surface of the cartridge 170 is in a circular shape or a polygonal shape. However, the main function of the cartridge 170 is to easily suction and lift the target part. Accordingly, the cross-sectional surface of the suctioned part is not limited to be in the circular shape or the polygonal shape.

According to an embodiment, the needle 120 is at least partially coated with an insulating substance, and is inserted into the cartridge 170 and fixed to the cartridge 170. For example, the needle 120 changes the target part of the cartridge 170 to be in the vacuum status to form a fine hole in the target part lifted at the negative pressure.

According to an embodiment, the needle 120 is sharpened, includes metal to be hard, and includes a hard material, such as metal, or silicone material. The needle 120 may be formed by plating a conductive material with a non-conductive material, in a hollowed structure. At least a portion, which includes a distal portion, of the needle 120 may be coated with any one among parylene, Teflon, and ceramic. The details of the coating of at least a portion (including a distal end portion of the needle 120) of the needle 120 will be described below.

According to an embodiment, the size of the fine hole is equal to the thickness of the needle 120, and the depth of the fine hole is varied depending on an extent that the needle 120 is injected. In addition, when the needle 120 injected into the skin must get out of the skin within a specific time in order to prevent an accident and reduce a pain.

According to an embodiment, the plurality of needles 120 are divided into a positive pole and a negative pole and connected. In other words, a needle tip may be implemented in a bipolar type including a positive-pole needle and a negative-pole needle. The arrangement of the positive-pole needle and the negative-pole needle of the needle tip will be described below, and even the arrangement of the active region of each needle will be described below.

In addition, according to an embodiment, the plurality of needles may include active regions electromagnetically conducted, in equal numbers at the same position. For example, when the plurality of needles are injected in the status the skin surface is maintained flat, as at least one active region is provided at the same position in each needle, the active region is disposed at the same depth under the skin surface, such that the electric energy is provided at a specific depth layer.

In addition, the plurality of needles may include active regions electromagnetically conducted at different positions. According to an embodiment, the positions of the active regions may be varied, depending on the arrangement positions in two dimensions (on the same plane) of each needle to compensate for that the skin surface is not flat and is pressed by pressure applied by the needle 120, when the needle 120 is injected into the skin. In detail, considering that the plurality of needs are not injected to the same depth since the skin surface is pressed when the needle 120 is injected into the skin, the position of the active region may be adjusted depending on the position in which the needle is fixed to the needle fixing unit and the needle is injected, such that energy may be applied to the same depth from the skin surface. For example, since the central area of the needle fixing unit is more sagged when the needle is injected, the active region of the needle 120 disposed at the central area of the needle fixing unit becomes close to the distal end portion.

In addition, according to another embodiment, as active regions electromagnetically conducted are provided in different numbers at different positions of the plurality of needles, energy may be applied to the entire portion of the skin layer to be injected with the needle and treated. For example, a first needle and a second needle are provided to be adjacent to each other and connected with different polarities, and the first needle has one active region provided at the intermediate position of the first needle instead of the distal end portion of the first needle, and the second needle has active regions provided at positions lower than that of the active region of the first needle. In this case, as the active region of the first needle and the plurality of active regions of the second needle are electrically connected to each other (e.g., the electric field is formed), the electric energy may be provided throughout the wide area of the skin.

According to an embodiment, the casing 110 may receive the driving unit 140 therein. A left casing and a right casing may be coupled to each other through a bolt 113, an inserting member 114, a first coupling member 111, and a second coupling member 112, and may be connected to the connector 160 through the connection member 180. A third coupling member 115 and a fourth coupling member 116 may be coupled to each other while communicating with the connection member 180.

According to an embodiment, although not illustrated in the drawing, a controller controls the operation of the driving unit 140 or the plurality of needles 120. In other words, the controller damages skin tissue around a depth in which the active region is disposed, by applying current, when the needle is injected into the skin. In addition, the controller may control the needle to be injected into the skin by allowing the needle to protrude out of the cartridge 170, after the apparatus is placed in the status in which the needles do not protrude. Then, the controller controls the needle to be discharged after applying energy to the active region at a desired depth under the skin surface and

In addition, according to another embodiment, when directly controlling the pressure at the inside (e.g., the space between the needle fixing unit and the skin surface) of the cartridge 170, the controller may adjust a pneumatic pressure inside the cartridge 170 so that the inside of the cartridge 170 becomes in the negative pressure status when bringing the surface of the cartridge 170 into contact with the target part, or operates the needle 120 such that the needle 10 is injected into the uniform depth of the skin when the inside (that is, the space between the needle fixing unit and the skin surface) of the cartridge 170 becomes in the negative pressure status.

According to an embodiment, the electricity supply unit 150 generates energy to be transmitted to the plurality of needles 120.

In this case, current generated from the electricity supply unit 150 is used to increase a temperature at the depth (that is, the target part) of the active region, which is conducted electromagnetically under the skin surface, to the level in which the skin tissue is heated. In addition, the electricity supply unit 150 applies alternating current to the active region (or an active zone).

According to an embodiment, the driving unit 140 directly or indirectly applies a force to the cartridge 170 such that the plurality of needles 120 fixed to the cartridge 170 are injected into the skin.

For example, when the force is directly applied to the cartridge 170, the driving unit 140 is directly and fixedly attached to the cartridge 170, such that the plurality of needles 120 are injected into the skin through vertical motion. Meanwhile, when the force is indirectly applied to the cartridge 170, the driving unit 140 applies the force to the cartridge 170 by striking the cartridge 170, such that the cartridge 170 is moved down.

In this case, it is preferred that the driving unit 140 is driven by any one of electromagnetic force moving by an electric signal, hydraulic force, air pressure, and a solenoid valve.

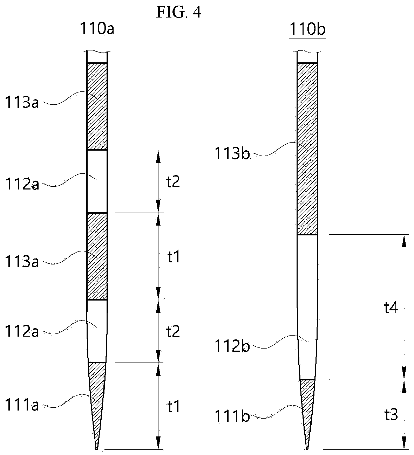

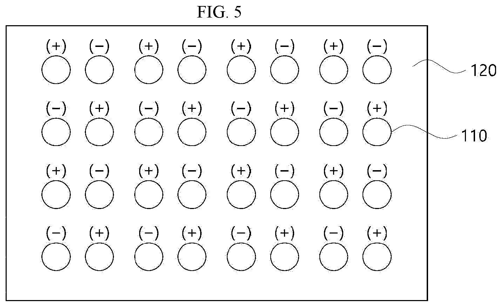

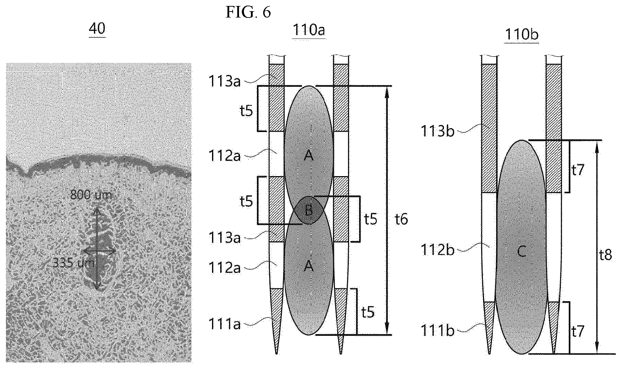

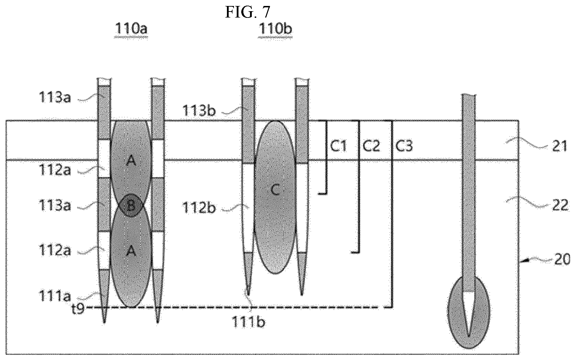

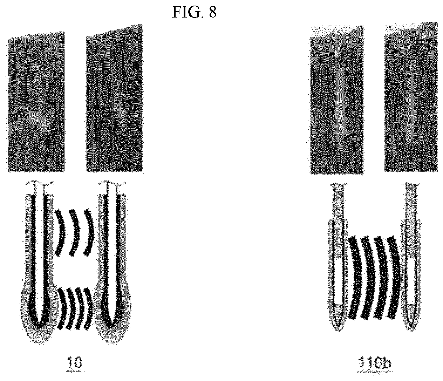

FIG. 4 is a view illustrating a needle, according to an embodiment of the inventive concept. FIG. 5 is a diagram illustrating an arrangement based on a polarity of a needle, according to an embodiment of the inventive concept. FIG. 6 is a view illustrating electric energy transmitted to skin by a needle, according to an embodiment of the inventive concept. FIG. 7 is a view illustrating a needle suitable for each skin depth, according to an embodiment of the inventive concept. FIG. 8 is a view illustrating an effect of transmitting electric energy by a needle, according to an embodiment of the inventive concept.

Referring to FIGS. 4 to 8, the needle 110 may include distal end portions 111a and 111b coated with an insulator, active regions 112a and 112b which are not coated with the insulator and electromagnetically conducted, and insulating regions 113a and 113b coated with the insulating substance. The first needle 110a and the second needle 110b illustrated in FIG. 4 will be described below by way of example for the convenience of explanation.

According to an embodiment, the distal end portions 111a and 111b may be coated with an insulator. As the distal end portions 111a and 111b of the needle 110 are insulated, the conventional damage of the tissue in the bell shape may be prevented. In other words, the insulating coating may offset the electric field resulting from the electric energy applied to the needle 110.

According to an embodiment, as a partial region of the needle 110 except for a distal end portion of the needle 110 is not insulated, the active regions 112a and 112b that are electromagnetically conducted may be formed on the needle 110. In other words, each needle 110 may include at least one active region 112a or 112b.

According to an embodiment, as illustrated in FIG. 5, the plurality of needles 110 having the same active regions 112a and 112b that are electromagnetically conducted may be disposed in the cartridge 170 in two dimensions. In other words, only a plurality of first needles 110a may be disposed or only a plurality of second needles 110b may be disposed.

According to an embodiment, the driving unit 140 or the controller 130 may apply current to reach the target depth after the needle 110a is injected into the skin.

According to an embodiment, the needle 110 may include the active regions 112a and 112b that are electromagnetically conducted and spaced apart from each other. In this case, electric energy may be applied to a plurality of skin parts corresponding to the depths of the skin in which the active regions 112a and 112b are positioned. In other words, as the plurality of active regions 112a and 112b are formed on the needle 110, the electric energy may be applied to the plurality of skin parts.

According to an embodiment, mutually different electrodes may be connected to the plurality of needles 110 in each row and each column. For example, as illustrated in FIG. 5, an electrode having a positive polarity and an electrode having a negative polarity may be alternately connected to the needles 110 in each row. An electrode having a positive polarity and an electrode having a negative polarity may be alternately connected to the needles 110 in each column. In this case, mutually different polarities may be disposed at an upper portion, a lower portion, a left portion, and a right portion from one electrode. In other words, as the needles 110 are arranged in the bipolar type in which mutually different polarities are alternately arranged, an electric field is formed with four negative polarities to corresponding to the upper portion, the lower portion, the left portion, and the right portion at a specific positive polarity. Accordingly, electric energy may be applied only skin part corresponding the specific depth of the skin.

According to another embodiment, at least two electrodes among adjacent electrodes of the plurality of needles 110 may have the same polarity. For example, differently from FIG. 5, one electrode having the same polarity may be disposed at one or more of the upper portions, the lower portion, the left portion, and the right portion of another electrode. For example, differently from FIG. 5, one electrode having the same polarity may be disposed at one or more of the upper portion, the lower portion, the left portion, and the right portion of another electrode.

According to an embodiment, when the needle 110 includes the plurality of active regions 112a and 112b electromagnetically conducted, electric energy may be transmitted to skin part including a part between the active regions 112a and 112b by adjusting current intensity.

According to an embodiment, the depth of the skin, in which the active regions 112a and 112b are disposed, may be adjusted, as the depth of the skin, in which the needle 110 is injected, is adjusted by the cartridge 170 of the apparatus for treating the skin of FIG. 2, the controller 130, or the driving unit 140 of FIG. 3.

According to an embodiment, needles 110 having the active regions 112a and 112b disposed at different positions are replaced and used to treat a specific depth of skin.

The details of the features of the needle 110 described above will be described below in detail. The needle 110 includes the active regions 112a and 112b, which correspond to the non-insulated parts and are electromagnetically conducted, to transmit the electric energy to skin part positioned at a specific depth of the skin. In addition, if necessary, the active regions 112a and 112b may be separated and formed. Accordingly, energy may be applied to a plurality of skin parts positioned at a specific depth of the skin.

Meanwhile, an energy transmission region (a region in which an electric field is formed) may be differently determined depending on the strength of electric energy received in the active region 112a and 112b which are electromagnetically conducted. For example, when a strong electric energy is received, an electric field is formed while covering the space between the plurality of active regions 112a and 112b. In other words, as illustrated in FIG. 6, the active region 112a formed at a lower portion of the needle 110a and the active region 112a formed at an upper portion of the needle 110a may transmit the electric energy to an upper region 'A' and a lower region 'A'. When the strength of the electric energy is strong, a partial region (overlap region) 'B' may be formed. In addition, to the contrary, when the strength of the electric energy is weak, the overlap region 'B' may not be formed, which is different from FIG. 6, and the first needle 110a may form the electric field only in the area adjacent to each of the active regions 112a and 112b.

According to an embodiment, in the needle 110, the coating lengths of the distal end portions 111a and 111b, the length and the number of the active regions 112a and 112b which are electromagnetically conducted, and the length and the number of the insulating regions 113a and 113b may be determined depending on the depth of the skin to be treated and relevant disease.

According to an embodiment, the distal end portions 111a and 111b, the active regions 112a and 112b which are electromagnetically conducted, and the insulating regions 113a and 113b are sequentially positioned. For example, the active regions 112a and 112b, which are electromagnetically conducted, and the insulating regions 113a and 113b may be formed in plural on the needle 110. The active regions 112a and 112b and the insulating regions 113a and 113b may be positioned while crossing each other.

According to an embodiment, the length of the distal end portions 111a and 111b coated with the insulator may be in the range of 0.2 mm to 0.3 mm. When the distal end portions 111a and 111b are coated with the insulator by the length in the range of 0.2 mm to 0.3 mm, the electric energy may be prevented from being intensively applied to the distal end portions 111a and 111b.

According to an embodiment, the lengths and the number of the active regions 112a and 112b, which are electromagnetically conducted, may be selected based on the depth of the skin requiring treatment.

Meanwhile, referring to FIG. 5, since the needles 110 to which different polarities are supplied are alternately arranged in a matrix form, electric energy may be intensively transmitted only to the depth of the skin positioned between the needles 110. In more detail, the needle 110 installed in the needle fixing unit 170 may include a needle to which electric energy having a positive polarity is supplied and a needle to which electric energy having a negative polarity is supplied. In other words, the needle to which electric energy having the positive polarity is supplied and the needle to which electric energy having the negative polarity is supplied may be alternately arranged. For example, an electric field may be formed between the needle to which electric energy having the positive polarity is supplied and the needle to which electric energy having the negative polarity is supplied, in the bipolar manner. Accordingly, as the electric energy may be applied to the desired depth range of the skin, tissue regeneration such as collagen may be induced, and the electric energy may be intensively applied only to the skin part corresponding to the depth of the skin adjacent to the active regions 112a and 112b. Accordingly, the damage to the tissue of the undesired region may be prevented.

According to an embodiment, as illustrated in FIG. 4, the first needle 110a may include two active regions 112a, the length 't1' of the distal end portion 111a coated with the insulator may be in the range of 0.28 mm to 0.32 mm, and the length 't2' of the active region 112a may be in the range of 0.23 mm to 0.27 mm. The length 't1' of the insulating region 113a positioned between the active regions 112a may be in the range of 0.28 mm to 0.32 mm. The insulating region 113a positioned at an upper portion of the drawing and occupying a remaining portion of the first needle 110a is not limited to the specific length.

According to an embodiment, as illustrated in FIG. 4, the second needle 110b may include one active region 112b. The length 't3' of the distal end portion 111b coated with the insulator may be in the range of 0.18 mm to 0.22 mm, and the length 't4' of the active region 112b may be in the range of 0.48 mm to 0.52 mm. The insulating region 113b positioned at an upper portion of the drawing and occupying a remaining portion of the second needle 110b is not limited to the specific length.

The first needle 110a and the second needle 110b have the above numeric value range for the following reasons to be described below with reference to FIGS. 6 and 7. As illustrated in the photograph located on the left side of FIG. 6, it may be recognized that, when the needle 110 is actually injected into the skin, energy is diffused to the length in the range of about 0.23 mm to about 0.25 mm in a vertical direction from the lengths of the active region 112a and 112b. In other words, the length 't5' of the region 'A', in which the energy is diffused in the vertical direction of the active region 112a of the first needle 110a, may be in the range of 0.23 mm to 0.25 mm. The length 't7' of the region 'C', in which the energy is diffused in the vertical direction of the active region 112b of the second needle 110a, may be in the range of 0.23 mm to 0.25 mm In other words, when the lengths of the distal end portion 111a and 111b, the active regions 112a and 112b, and the insulating regions 113a and 113b are determined based on the lengths of the regions 'A', 'B', and 'C' in which the energy is diffused, as illustrated in FIG. 5, the length 't6' of the energy transmission region of the first needle 110a may be in the range of 1.25 mm to 1.32 mm, and the length 't8' of the energy transmission region of the second needle 110b may be in the range of 0.98 mm to 1.02 mm. Actually, as illustrated in FIG. 7, the depth 'C1' of the epidermis-base skin to transmit electric energy to treat freckles may be in the range of 0 mm to 0.5 mm, and the depth 'C2' of the epidermis-to-skin to transmit electric energy for skin tone, skin texture, and skin tightening may be in the range of 0 to 1 mm. The depth 'C3' of the epidermis-to-skin to transmit electric energy to treat pores and Rosacea may be in the range of 0 to 1. 25 mm. The depth of skin for transmitting the electric energy may be varied depending on the target (e.g., freckles) of skin to be treated. According to the inventive concept, the first needle 110a and the second needle 110b may be configured to transmit electric energy depending on the target of the skin to be treated. In other words, the first needle 110a may transmit electric energy to improve freckles, skin tone, skin texture, tightening, pores, and Rosacea, and the second needle 110b may transmit electric energy to improve freckles, skin tone, skin texture, and tightening.

In other words, as described above, the number and length of the active regions 112a and 112b may be appropriately selected such that electric energy is transmitted only to the depth of a specific skin in the needle 110 of the inventive concept. In addition to the number and the length of the active regions 112a and 112b, the controller 140 described above may adjust the strength of the electric energy, which is applied to the needle 110 to adjust the size of a region in which electric energy is transmitted to the depth of the skin. In other words, as the strength of the electric energy is adjusted, the overlap region 'B' is present or absent.

Meanwhile, as described above, according to the needle 110 of the inventive concept, electric energy having opposite polarities are alternately supplied to the needles as illustrated in FIG. 5. Accordingly, the electric energy is uniformly transmitted to the skin between the needles. In addition, as the distal end portion is coated with the insulator, the electric energy is not intensively applied to the distal end portion. Accordingly, as recognized in a left photograph of FIG. 8, electric energy is intensively applied to the distal end portion and is not uniformly transmitted to the skin in the direction in which the needle is injected into the skin, in the existing needle 10. To the contrary, according to the needle 110b of the inventive concept, the electric energy is uniformly transmitted in the direction in which the needle is injected into the skin, without being intensively applied to the distal end portion, which is recognized from a right photograph of FIG. 8.

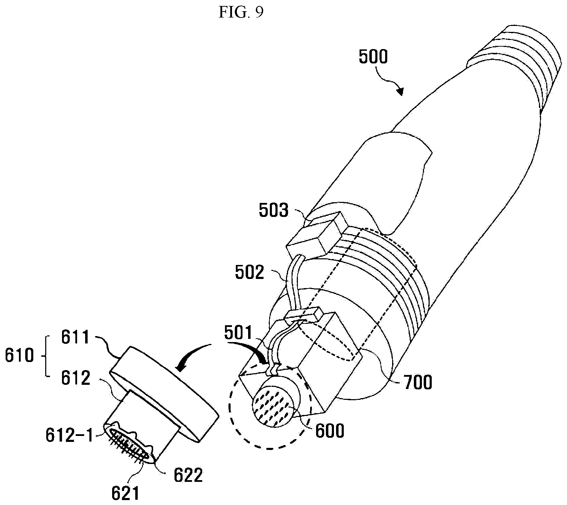

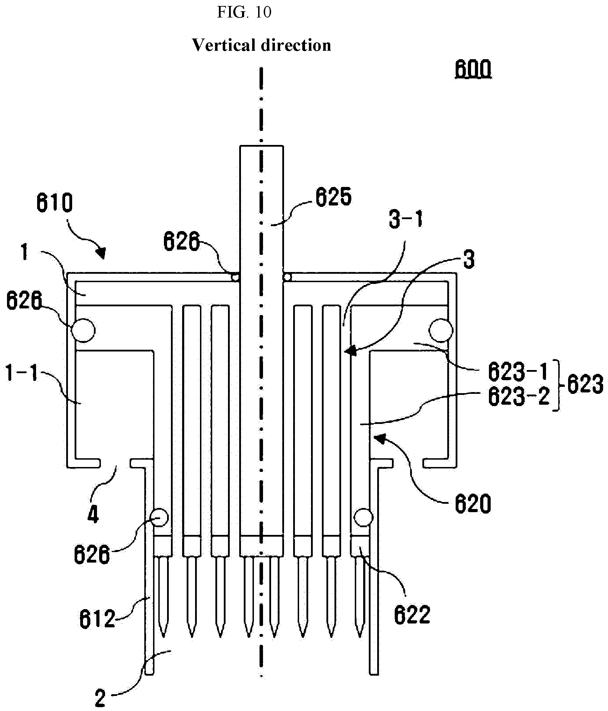

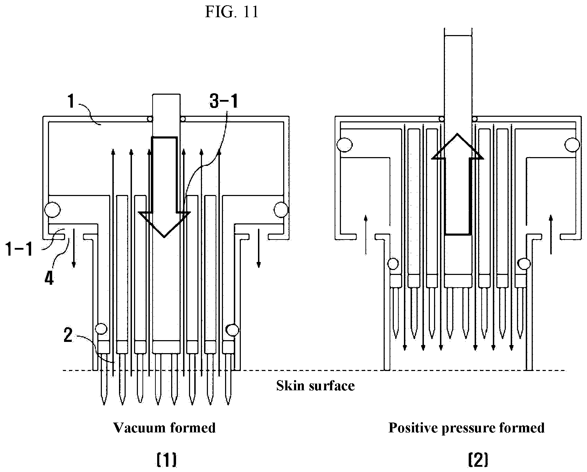

FIG. 9 is a perspective view illustrating a handpiece, according to an embodiment of the inventive concept. FIG. 10 is a cross-sectional view of a needle tip for applying current, according to an embodiment of the inventive concept. FIG. 11 is a cross-sectional view of a needle tip for applying current, which operates to produce a pumping effect, according to an embodiment of the inventive concept.

Referring to FIGS. 9 and 11, according to the inventive concept, an apparatus for treating skin may include a handpiece 500.

The handpiece 500 is gripped by a doctor. The doctor brings the handpiece 500 in contact with skin of a subject while moving the handpiece 500 to change a target point (e.g., a part of a face). The handpiece 500 may be connected to the apparatus for treating skin through a cable.

A driving module 700 and a power module may be embedded in the handpiece 500. Accordingly, the cable may electrically connect each of the driving module 700 and the power module embedded in the handpiece 500 to an electronic control module embedded in the apparatus for treating skin. A needle tip 600 for applying current may be mounted on a distal end portion of the handpiece 500. In this case, the needle tip 600 for applying the current may be mounted on the distal end portion of the handpiece 500 to be replaceable in the form of a cartridge.

A first conductive member 501, which electrically connects the needle unit 620 of the needle tip 600 for applying the current to the power module, and a second conductive member 502, which is docked on a cable connector 503 to electrically connect the power module to the cable may be disposed outside the handpiece 500. In this case, the first conductive member 501 and the second conductive member 502 may be manufactured in a film form. For example, the first conductive member 501 and the second conductive member 502 may be flexible printed circuit boards (FPCB). As described below, the needle unit 620 of the needle tip 600, which is for applying the current, moves (drives) to reciprocate. Accordingly, to prevent a conductive line from interfere with the needle unit 620, the needle unit 620 of the needle tip 60 for applying current, a conductive line of the power module, a conductive line of a cable are provided at an outer portion of the handpiece 500.

The needle tip 600 for applying the current may be a member which applies a radio frequency to the skin depth at the target point. The needle tip 600 for applying the current may be detachably mounted on the distal end portion of the handpiece 500. In other words, according to the inventive concept, the needle tip 600 for applying the current may be manufactured in the form of a cartridge and may be replaceable with new one.

The needle tip 600 for applying the current may include a cylinder 610 and a needle unit 620. The cylinder 610, which serves as a 'fixture', may be a member detachably mounted on a distal end portion of the handpiece 500. The needle unit 620, which serves as a 'mover (which moves in the vertical direction), may include at least one needle 621 to invade the skin deep part at a target point at a specific period (a driving period of the driving module), and may apply a radio frequency (RF) to a dermis layer of the skin if necessary.

The cylinder 610 may be hollowed in the vertical direction. The needle unit 620 may be disposed in the inner space of the cylinder 610. The bottom surface of the cylinder 610 may be open, and a lower end of the cylinder 610 may be disposed on the skin surface at the target point. Accordingly, the open portion of the cylinder 610 may be closed by the skin surface at the target point.

The cylinder 610 may include a first cylinder 611 and a second cylinder 612. In this case, the first cylinder 611 may be positioned at an upper portion, and the second cylinder 612 may be located at a lower portion. A lower end of the first cylinder 611 may be connected to an upper end of the second cylinder 612. A bottom surface of the second cylinder 612 may be open.

The needle unit 620 may be disposed inside the first cylinder 611 and the second cylinder 612, and a connection portion between the first cylinder 611 and the second cylinder 612 may be closed by the needle unit 620.

A connecting rod 625 of the needle unit 620 may pass through a top surface of the first cylinder 611. A first space 1 and an extra space 1-1 may be formed in the first cylinder 611 by a first plunger 623-1 of the needle unit 620. In other words, the inner space of the first cylinder 611 may be closed in the vertical direction by the first plunger 623-1 of the needle unit 620, and may be divided into the first space 1 positioned at an upper portion and the extra space 1-1 positioned at a lower portion.

To maintain airtightness of the first space 1 of the first cylinder 611, a gasket 626 may be interposed between the top surface of the first cylinder 611 and the connecting rod 625 of the needle unit 620. In addition, the gasket 626 may be interposed between an inner circumferential surface of the first cylinder 611 and an outer circumferential surface of a first plunger 623-1 of the needle unit 620.

The lower end of the second cylinder 612 may be disposed on the surface of the skin at the target point. Accordingly, the open portion of the bottom surface of the second cylinder 612 may be closed by the skin surface at the target point. A second space 2 having an open bottom surface may be formed in the second cylinder 612 by a second plunger 623-2 of the needle unit 620. The inner space of the second cylinder 612 may be closed in the vertical direction by the second plunger 623-2 of the needle unit 620, a holder 622 of the needle unit 620 and a second plunger 623-2 of the needle unit 620 may be disposed at an upper portion of the second cylinder 612, and the second space 2 having an open bottom surface may be positioned at a lower portion of the second cylinder 612.

At least one groove 612-1 may be formed in the bottom surface of the second cylinder 612 (see FIG. 3). The at least one groove 612-1 of the second cylinder 612 may be formed from the outer circumferential surface of the second cylinder 612 to the inner circumferential surface of the second cylinder 612. In other words, the at least one groove 612-1 of the second cylinder 612 may be formed through the second cylinder 612. In addition, the at least one groove 612-1 of the second cylinder 612 may be arranged to be spaced apart from each other along the circumference of the bottom surface of the second cylinder 612. The at least one groove 612-1 of the second cylinder 612 may be formed to be spaced apart from each other in the circumferential direction.

Meanwhile, the open portion of the bottom surface of the second cylinder 2 may be closed by the skin surface at the target point. In this case, to maintain airtightness of the second space 2, the gasket 626 may be disposed between the inner circumferential surface of the second cylinder 612 and the outer circumferential surface of the second plunger 623-2 of the needle unit 620. Meanwhile, in the status in which the second space 2 maintains airtightness, only the lower end portion is selectively connected to the outside by the at least one groove 612-1 of the second cylinder 612 to increase a pumping effect to be described later.

At least one needle 621 of the needle unit 620 may be disposed in the second space 2. As described above, since the bottom surface of the second space 2 is open, the at least one needle 621 may be introduced into the skin surface at the target point through the open portion of the second space 2.

The cross-sectional area perpendicular to the vertical direction of the first cylinder 611 may be wider than the cross-sectional area perpendicular to the vertical direction of the second cylinder 612. Accordingly, the volume variation of the first space 1 of the first cylinder 611 may be greater than the volume variation t of the second space 2 of the second cylinder 612, as the plunger 623 of the needle unit 623 reciprocates in the vertical direction.

The cylinder 610 may further include a seat 613 (see FIG. 11). The seat 613 may be positioned in the second space 2. The seat 613 may be disposed to be inclined downward inward of the inner circumferential surface of the second cylinder 612. The seat 613 may have a ring shape and may be disposed along the inner circumferential surface of the second cylinder 612. In this case, according to the inventive concept, the seat 613 may be disposed along the circumference of the at least one needle 621 of the needle unit 620, which is similar to the shape of the "valve seat". In other words, the seat 613 may cover the circumference of the at least one needle 621 of the needle unit 620.

An outer end portion of the seat 613 may be a fixed end, and an inner end portion of the seat 613 may be a free end. Accordingly, the downward-inclined angle of the seat 613 may be varied by the flow of air around the seat 613. To improve the variable action of the inclination angle, the seat 613 may be formed of an elastic material.

The outer end portion of the seat 613 may be disposed above the at least one groove 612-1 of the second cylinder 612. Accordingly, the inclination angle of the seat 613 may be varied depending on the flow of air flowing through the at least one groove 612-1 of the second cylinder 612. The seat 613 may interact with the at least one groove 612-1 of the second cylinder 612 to increase a pumping effect to be described later.

The needle unit 620 may be disposed inside the cylinder 610. The needle unit 620 may reciprocate in the vertical direction by the driving module 700. In other words, the needle unit 620 may be disposed inside the first cylinder 611 and the second cylinder 612, and may reciprocate like a "Piston". Furthermore, the plunger 623 may be provided to partition the inner space of the first cylinder 611 and the second cylinder 612, and the volume of the inner space of the first cylinder 611 and the second cylinder 612 may be changed.

The needle unit 620 may repeatedly (periodically) invade the skin at the target point, as the needle unit 620 reciprocates in the vertical direction. Furthermore, the needle unit 620 generates a radio frequency at the skin depth of the target point, and collagen and elastic fibers damaged by thermal energy due to the radio frequency are regenerated over time, thereby increasing skin elasticity.

The needle unit 620 may include the at least one needle 621, the holder 622, the plunger 623, and the connecting rod 625.

The at least one needle 621 may reciprocate together with the plunger 623 to be injected into the skin and discharged from the skin alternately. A radio frequency may be applied to the at least one needle 621 to generate thermal energy at the skin depth of the target point.

However, the inventive concept is limited thereto, electric energy having various wavelength bands and an ultrasonic wave may be applied to the at least one needle 621. Further, as described above, the electric energy or the ultrasonic wave may not be applied to the at least one needle 621.

When the electric energy having the radio frequency is applied to the at least one needle 621, the at least one needle 621 is electrically connected to the power module to receive power. To this end, the at least one needle 621 may be electrically connected to the power module by the first conductive module 501 described above.

Meanwhile, the at least one needle 621 may be a bipolar type electrode unit in which the plurality of electrodes have two polarities and thus a radio frequency is generated between neighboring electrodes, or a monopolar type in which a plurality of electrodes has the same polarity. Meanwhile, when the at least one needle 621 is in the monopolar type, a ground electrode module (not shown) to reflux the radio frequency generated from the at least one needle 621 may be further provided.

The at least one needle 621 may be supported by the holder 622. The at least one needle 621 may extend downward from the holder 622. The at least one needle 621 may be disposed in the second space 2 of the second cylinder 612

The at least one needle 621 may reciprocate in the vertical direction by the driving module 700. The lower end portion of the at least one needle 621 may be disposed at the skin depth of the target point, at a bottom dead point of the needle unit 620. The lower end portion of the at least one needle 621 may be disposed on the skin surface of the target point, at the top dead point of the needle unit 620.

Accordingly, the at least one needle 621 may repeatedly invade into the skin depth of the target point. In this case, the at least one needle 621 protrudes downward through the open portion formed in the bottom surface of the second space 2 of the second cylinder 612 and may recover (return) upward. Meanwhile, the invasion depth of the at least one needle 621 may be approximately 2.1 mm.

The holder 622 may be a member to support the at least one needle 621. The holder 622 may be disposed in the second space 2 of the second cylinder 612, which is similar to the at least one needle 621. In addition, the holder 622 may be disposed on a bottom surface of the second plunger 623-2 to be coupled to the second plunger 623-2. Furthermore, in some cases, the holder 622 may be omitted. In this case, the at least one needle 621 may be directly disposed on the plunger 623.

The plunger 623 may reciprocate downward and upward to form the first space 1 and the second space 2 inside the cylinder 610. In addition, a first channel 3, which connects the first space 1 to the second space 2, may be formed in the plunger 623.

The variation in the volume of the first space 1 is greater than the variation in the volume of the second space 2, due to the reciprocating movement of the plunger 623. Accordingly, the gas in the second space 2 may move to the first space 1 through the first channel 3, when the plunger 623 moves downward, and the gas in the first space 1 may move to the second space 2 through the first channel 3, when the plunger 623 moves upward

Accordingly, when the plunger 623 moves downward, the at least needle 621 may invade the skin so that, and a negative pressure status may be formed (pressure decreased) in the second space 2. When the plunger 623 moves upward, the at least needle 621 may be discharged from the skin so that and a positive pressure status may be formed (pressure increased).

The plunger 623 may include a first plunger 623-1 and a second plunger 623-2. The first plunger 623-1 may be disposed in the inner space of the first cylinder 611. The first plunger 623-1 may close the inner space of the first cylinder 611 in the vertical direction to form the first space 1 positioned at the upper portion of the first cylinder 611 and the extra space 1-1 positioned at the lower portion of the first cylinder 611.

The first plunger 623-1 may reciprocate in the vertical direction by driving force of the driving module 700. When the first plunger 623-1 moves downward, the volume of the first space 1 may be increased, and the volume of the extra space 1-1 may be reduced (see reference number 1 of FIG. 5). When the first plunger 623-1 moves upward, the volume of the first space 1 may be reduced, and the volume of the extra space 3 may be increased (see reference number 2 of FIG. 5).

The second plunger 623-2 may be disposed in the inner space of the second cylinder 612. The second plunger 623-2 may close the inner space of the second cylinder 612 in the vertical direction to form the second space 2 in the second cylinder 612.

The second plunger 623-2 may reciprocate in the vertical direction by driving force of the driving module 700. When the second plunger 623-2 moves downward, the volume of the second space 2 may be reduced (see reference number 1 of FIG. 5). When the second plunger 623-2 moves upward, the volume of the second space 2 may be reduced (see reference number 2 of FIG. 5).

In addition, the first channel 3, which connects the first space 1 to the second space 2, may be formed in the first plunger 623-1 and the second plunger 623-2. In this case, the first channel 3 formed in the first plunger 623-1 and the second plunger 623-2 may be at least one fluid passage 3-1 (formed in the first plunger 623-1 and the second plunger 623-2) which passes through the first plunger 623-1 and the second plunger 623-2 in the vertical direction.

The connecting rod 625 may be disposed at the upper portion of the first plunger 623-1. The connecting rod 625 may move in the vertical direction by the driving force of the driving module 700. The connecting rod 625 may be connected to the driving module 700 and the first plunger 623-1 to perform a function of transmitting the driving force of the driving module 700 to the first plunger 623-1.

Hereinafter, an operation (pumping effect) of the needle tip 600 for applying the current will be described with reference to FIG. 11. When the apparatus for treating skin of the inventive concept is operated, the needle unit 620 reciprocates in the vertical direction (up and down direction), and may repeatedly invade the skin at a target point (when the radio frequency is applied, thermal energy is generated at the skin depth). Meanwhile, a drug may be applied to the skin surface at the target point to relieve pain due to invasion or to promote the regeneration of the wound.

When the needle unit 620 moves downward, the volume of the first space 1 may be increased, and the volume of the second space 2 may be decreased. In this case, due to the difference in the cross-sectional area perpendicular to the vertical direction of the first space 1 and the second space 2, the variation in the volume of the first space 1 may be greater than the variation in the volume of the second space 2. In other words, an increase in the volume of the first space 1 may be greater than a decrease in the volume of the second space 2. Meanwhile, since the first space 1 and the second space 2 are connected to each other by the first channel 3, the gas in the second space 2 may move to the first space 1 through the first channel 3 (due to a pressure difference resulting from the variation in volume, see reference number 1 of FIG. 11). Accordingly, the second space 2 becomes in a "negative pressure status (pressure reduced; to the contrary, the first space becomes in a positive pressure status due to pressure increased)" to suction the skin surface at the target point, and to equalize the height of the skin surface at the target point. Therefore, the at least one needle 621 may invade the skin at a uniform depth (effect of invading the skin at an equal depth, because all of the plurality of needles irradiate radio frequencies at an invasive depth (a preset depth) that meets medical design conditions).

When the needle unit 620 moves upward, the volume of the first space 1 may be increased, and the volume of the second space 2 may be decreased. In this case, due to the difference in the cross-sectional area perpendicular to the vertical direction of the first space 1 and the second space 2, the variation in the volume of the first space 1 may be greater than the variation in the volume of the second space 2. In other words, a decrease in the volume of the first space 1 may be greater than an increase in the volume of the second space 2 of the second cylinder 612. Meanwhile, since the first space 1 and the second space 2 are connected to each other by the first channel 3, the gas in the first space 1 may move to the second space 1 through the first channel 3 (due to a pressure difference resulting from the variation in volume; see reference number 2 of FIG. 5). Accordingly, the second space 2 becomes in a "positive pressure status (pressure increased; to the contrary, the first space becomes in a negative pressure status due to pressure decreased), such that the drug applied to the skin surface at the target point to be deeply injected into an invaded part (a hole formed as the needle electrode is injected and discharged).

Meanwhile, a second channel 4 may be formed in the first cylinder 611 to connect the extra space 1-1 to the outside. The second channel 4 may prevent the pressure of the gas in the extra space 1-1 from interfering with the reciprocating movement of the first plunger 623-1 in the vertical direction. In other words, when the needle unit 620 moves downward, the air in the surplus space 1-1 may be discharged to the outside to remove resistance.

According to an embodiment, the needle 110 of the inventive concept may be manufactured as follows. First, silicone having a thickness for forming the active regions 112a and 112b corresponding to the non-insulated parts may be prepared. The needle 110 may be coupled to the cartridge 170, and may be inserted into the silicone such that the needle 110 reaches positions for forming the active regions 112a and 112b. In the status that the needle 110 is inserted in the silicone, the insulating material is sprayed to form the distal end portions 111a and 111b and the insulating regions 113a and 113b. Meanwhile, the needle 110 is manufactured such that the needle 110 is not insulated partially. Alternatively, the needle 110, which is completely manufactured, may be inserted into a silicon layer such that insulating treatment is performed.

In detail, when needle tips including active regions, which are formed in equal numbers at the same specific positions, are manufactured, a needle, which is not insulated, is first fixed into the cartridge 170 and is inserted into silicone having the thickness corresponding to the area range of the active region. In this case, a silicone pad having a specific thickness may be pulled bidirectionally to be prevented from sagged when the needle is inserted, such that the plurality of needles are simultaneously disposed at deep parts. In addition, when the plurality of active regions are formed at the same positions of the needles, the needles are inserted into the silicone pads having the thickness corresponding to the area range of the active regions, in the status that the silicone pads are maintained by a distance between the active regions. Thereafter, an insulating treatment is performed with respect to the needles, in the status that the silicone is disposed at the position in which the active region is formed. Accordingly, the plurality of needles may have the active regions at the same positions.

In this case, the silicone may be substituted with various materials, as long as the materials are disposed at a desired position of the needle, as the needle is inserted into the materials, and are formed at a thickness corresponding to an area range of the active region. In other words, the silicone may be substituted with all materials sufficient to prevent an insulating material from being sprayed on the surface of the active region, as at least one needle is inserted into the silicone, and the silicone is disposed on the needle. For example, a flexible material, such as silicone or rubber, may be used.

According to an embodiment of the inventive concept, a needle tip for applying current may include a needle fixing unit, and a plurality of needles disposed in two dimensions in the needle fixing unit, each needle may include at least one active region which is formed as an insulator is not coated on a partial area of the needle except for a distal end portion of the needle. The plurality of needles may be connected to electrodes having alternately different polarities, and four needles positioned around a needle having positive polarity may have negative polarity. When the plurality of needles may include a plurality of active regions spaced apart from each other, the electric energy may be transmitted between the plurality of active regions spaced apart from each other by adjusting the intensity of a current applied to each of the electrodes of the plurality of needles.

According to an embodiment of the inventive concept, the apparatus for treating skin includes a casing to suction a target part of skin surface, a plurality of needles of claim 1, which are disposed inside the casing, a needle fixing unit to fix the plurality of needles of claim 1 inserted, a controller to control an operation of the casing and operations of the plurality of needles of claim 1, and an electric energy transmitting unit electrically connected to the plurality of needles of claim 1 to transmit electric energy, and an electric energy generating unit to generate the electric energy to be transmitted to the plurality of needles of claim 1 through the electric energy transmitting unit.

According to an embodiment of the inventive concept, the handpiece may have a needle tip for applying current according to an embodiment of the inventive concept, which is mounted in the handpiece

In addition, according to an embodiment, a method for manufacturing a needle tip for applying current includes preparing silicone having a thickness for forming an active region corresponding to a non-insulated part, coupling a plurality of needles to a needle fixing unit, inserting the plurality of needles into the silicon to positions of the plurality of needles, which are used for forming the active region, and forming a distal end portion and an insulating area by spraying the insulating material in the status that the plurality of needles are inserted into the silicone.

Although the embodiment of the inventive concept have been described with reference to accompanying drawings, those skilled in the art should understand that various modifications are possible without departing from the technical scope of the inventive concept or without changing the technical sprite or the subject matter of the inventive concept. Therefore, those skilled in the art should understand that the technical embodiments are provided for the illustrative purpose in all aspects and the inventive concept is not limited thereto.

## Claims

1. A needle tip for applying current, the needle tip comprising:
a needle fixing unit; and
a plurality of needles disposed on one surface of the needle fixing unit, wherein each needle includes at least one active region which is formed, as an insulator is not coated on a partial area of the needle except for a distal end portion of the needle and is electromagnetically conducted,
wherein the plurality of needles are connected to electrodes having alternately different polarities.

2. The needle tip of claim 1, wherein when the plurality of needles include a plurality of active regions spaced apart from each other,
electric energy is transmitted between the plurality of active regions spaced apart from each other, by adjusting intensity of current applied to each of the electrodes connected to the plurality of needles.

3. The needle tip of claim 1, further comprising:
a casing making contact with a skin surface to form a first space between the skin surface and the needle fixing unit, and to move such that the needle fixing unit injects the needle into skin and discharges the needle out of the skin,
wherein the first space has a negative pressure formed before the needle is injected into the skin.

4. The needle tip of claim 1, wherein the needle tip is connected to a handpiece which moves the needle fixing unit and used, and
wherein the at least one active region of the plurality of needles, which is electromagnetically conducted, is determined in position inside a skin by a control of a driving unit provided in the handpiece.

5. The needle tip of claim 4, wherein the plurality of needles include at least one active region, which is electromagnetically conducted, has an equal size, and is positioned at the same position of the needles except for the distal end portion of each needle, and
wherein the needle tip heats a skin tissue at a depth in which the active region is disposed, as the needle is injected into the skin.

6. The needle tip of claim 4, wherein the plurality of needles include at least one active region, which is electromagnetically conducted, has an equal size, and is positioned at the same position of the needles except for the distal end portion of each needle, and
wherein the needle tip heats a tissue in a region of the skin, in which the plurality of active regions electromagnetically conducted are disposed, and a tissue in an entire area of the skin, which is interposed between the plurality of active regions electromagnetically conducted, as the needle tip is injected into the skin and current having a specific intensity or more is applied.

7. The needle tip of claim 1, wherein a needle adjacent to a needle, which is connected to an electrode having a positive polarity, of the plurality of needles is connected to an electrode having a negative polarity.

8. A handpiece including a needle tip mounted on a handpiece for applying current according to one of claim 1 to claim 7.

9. An apparatus for treating skin, the apparatus comprising:
a casing configured to suction a target part of a skin surface;
a plurality of needles, which are disposed inside the casing;
a needle fixing unit configured to fix the plurality of needles of claim 1 which are inserted;
a controller configured to control an operation of the casing and operations of the plurality of needles;
an electric energy transmitting unit electrically connected to the plurality of needles to transmit electric energy; and
an electric energy generating unit configured to generate the electric energy to be transmitted to the plurality of needles through the electric energy transmitting unit.

10. A method for manufacturing a needle tip for applying current according to any one of claim 1 to claim 7, the method comprising:
preparing a conductive material having a thickness for forming an active region corresponding to a non-insulated part;
coupling a plurality of needles to a needle fixing unit;
inserting the plurality of needles into the conductive material to positions of the plurality of needles, which are used for forming the active region; and
forming a distal end portion and an insulating area by spraying an insulating material, in a status that the plurality of needles are inserted into the conductive material.
